# EUROPÄISCHE PATENTSCHRIFT

(11) **EP 0 929 516 B1**
(45) Veröffentlichungstag und Bekanntmachung des Hinweises auf die Patenterteilung: **18.09.2002**
(21) Anmeldenummer: 97942007.2
(22) Anmeldetag: 09.09.1997
(51) Int. Cl.: C07C 257/18, C07C 257/20, C07C 271/64, A61K 31/155

(54) **BENZAMIDINDERIVATE UND IHRE VERWENDUNG ALS ARZNEIMITTEL MIT LTB4-ANTAGONISTISCHER WIRKUNG**
BENZAMIDINE DERIVATIVES AND THE USE THEREOF AS MEDICAMENTS WITH LTB4-ANTAGONISTIC EFFECT
DERIVES DE BENZAMIDINE ET LEUR UTILISATION COMME MEDICAMENTS AYANT UN EFFET ANTAGONISTE VIS-A-VIS DE LTB4

(30) Priorität: 10.09.1996 DE 19636689
(43) Veröffentlichungstag der Anmeldung: 21.07.1999
(60) Teilanmeldung: 02003690.1
(73) Patentinhaber: Boehringer Ingelheim Pharma KG, 55218 Ingelheim am Rhein (DE); Boehringer Ingelheim International GmbH, 55218 Ingelheim am Rhein (DE)
(72) Erfinder: SCHROMM, Kurt, D-55218 Ingelheim am Rhein (DE); ANDERSKEWITZ, Ralf, D-55411 Bingen (DE); RENTH, Ernst-Otto, D-24105 Kiel (DE); BIRKE, Franz, D-55218 Ingelheim am Rhein (DE); JENNEWEIN, Hans, Michael, D-65193 Wiesbaden (DE); MEADE, Christopher, John, Montague, D-55411 Bingen (DE)
(74) Vertreter: Laudien, Dieter, Dr.
(86) Internationale Anmeldenummer: EP9704921
(87) Internationale Veröffentlichungsnummer: WO98011062

(56) Entgegenhaltungen:
- EP-A- 0 496 378
- EP-A- 0 518 818
- EP-A- 0 574 808
- WO-A-93/16036
- DE-A- 4 424 713
- DE-A- 19 546 452

## Beschreibung

Die vorliegende Erfindung betrifft neue Benzamidinderivate, Verfahren zu ihrer Herstellung sowie ihre Verwendung als Arzneimittel. Die neuen Benzamidinderivate entsprechen der allgemeinen Formel 1 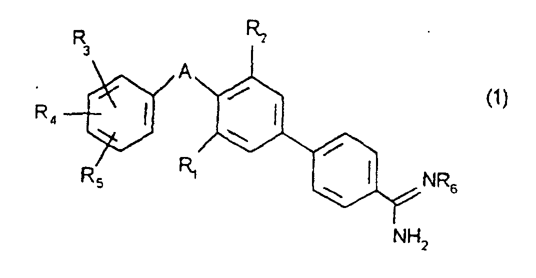 worin
- A: -OC₂-C₄-Alkylen-O- oder -C₁-C₃-Alkylen-O-
- R₁: C₁-C₆-Alkyl, verzweigt oder unverzweigt, C₃-C₆-Alkenyl, verzweigt oder unverzweigt, vorzugsweise Allyl oder F, Cl, Br, I;
- R₂: Wasserstoff, C₁-C₆-Alkyl, verzweigt oder unverzweigt, C₃-C₆-Alkenyl, verzweigt oder unverzweigt vorzugsweise Allyl oder F, Cl, Br, I;
- R₃ und R₄,: die gleich oder verschieden sein können, unabhängig voneinander Wasserstoff, C₁-C₆-Alkyl, verzweigt oder unverzweigt, C₃-C₆-Alkenyl, verzweigt oder unverzweigt, vorzugsweise Allyl, C₁-C₆-Alkoxy, (C₁-C₄-Alkyl)OC(O)O-, OH, CF₃ oder zusammen einen ankondensiertes ein- oder zweikerniges Ringsystem bilden können, das vollständig oder teilweise durchkonjugiert sein kann und gegebenenfalls ein oder zwei Heteroatome aus der Gruppe Sauerstoff, Schwefel oder Stickstoff aufweisen kann. die gleich oder verschieden sein können das gegebenenfal's durch OH, C₁-C₄-Alkoxy, C₁-C₄-Alkyl substituiert sein kann;
- R₅: Wasserstoff, Aryl, bevorzugt Phenyl, -OPhenyl, -CR₇R₈Phenyl, -COPhenyl, SO₂-Phenyl, -CH(OH)Phenyl wobei der Phenylring durch OH oder C₁-C₄-Alkoxy substituiert sein kann oder -C(O)NR₉R₁₀,
- R₆: Wasserstoff, C₁₋C₆-Alkoxycarbonyl, (C₁-C₅-Alkyl)-carbonyl oder -C(O)-O-(C1-C6-Alkylen)-NR₁₁R₁₂;
- R₇ und R₈,: die gleich oder verschieden sein können, Wasserstoff, C₁-C₈-Alkyl, verzweigt oder unverzweigt;
- R₉ und R₁₀,: die gleich oder verschieden sein können, unabhängig voneinander Wasserstoff, C₁-C₈-Alkyl, verzweigt oder unverzweigt;
- R₁₁ und R₁₂,: die gleich oder verschieden sein können, unabhängig voneinander Wasserstoff, C₁-C₈-Alkyl, verzweigt oder unverzweigt;
bedeuten können,
gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren.

Bevorzugt sind Verbindungen der allgemeinen Formel 1, in der
- A: -OCH₂CH₂O-, -CH₂O-, -CH₂CH₂CH₂O-;
- R₁: C₁-C₅-Alkyl, verzweigt oder unverzweigt, Allyl;
- R₂: Wasserstoff;
- R₃: Wasserstof, C₁-C₆-Alkyl, OCH₃, C₂H₅OC(O)O-, OH, Cl, F, CF₃;
- R₄: Wasserstoff, -OCH₃, OH;
- R₃ und R₄: zusammen einen ankondensierten Benzolring, ein 3,4-Dihydrocumarinsystem oder ein 1,3-Dioxolansystem, der bzw. das durch OH, C₁-C₃-Alkyl, OCH₃ substituiert sein kann;
- R₅: Wasserstoff, Phenyl, OPhenyl, -CR₇R₈Phenyl, wobei der Phenylring durch OH, OCH₃ substituiert sein kann, oder -C(O)NR₉R₁₀;
- R₆: Wasserstoff oder C₁-C₄-Alkoxycarbonyl oder -C(O)-O-(CH₂)₂-N(C₂H₅)₂;
- R₇ und R₈,: die gleich oder verschieden sein könen, unabhhängig voneinander Wasserstoff, CH₃ oder CF₃;
- R₉ und R₁₀,: die gleich oder verschieden sein können, Wasserstoff, C₁-C₈-Alkyl, verzweigt oder unverzweigt;
bedeuten können,

Soweit nicht im einzelnen abweichende Angaben gemacht werden, werden die allgemeinen Definitionen im folgenden Sinn gebraucht:
C₁-C₈-Alkyl, C₁-C₅-Alkyl und C₁-C₄-Alkyl steht im allgemeinen für einen verzweigten oder unverzweigten Kohlenwasserstoffrest mit 1 bis 4 oder 5 bzw. 8 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
   Methyl, Ethyl, Propyl, 1-Methylethyl (Isopropyl), n-Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 4 Kohlenstoffatomen, wie Methyl, Ethyl, Propyl, *iso*Propyl, n-Butyl, 1-Methylpropyl, 2-Methylpropyl oder 1,1-Dimethylethyl.

Entsprechend bedeutet Alkylen eine verzweigte oder unverzweigte zweibindige Kohlenwasserstoffbrücke mit 1 bis 8 Kohlenstoffatomen, die gegebenenfalls mit einem oder mehreren Halogenatom(en) - vorzugsweise Fluor - substituiert sein kann, die untereinander gleich oder verschieden sein können.

Aryl steht im allgemeinen für einen aromatischen Rest mit 6 bis 10 Kohlenstoffatomen - auch in Zusammensetzungen, wobei der Aromat mit einer oder mehreren Niederalkylgruppe(n), Trifluormethylgruppe(n), Cyanogruppe(n), Alkoxygruppe(n), Nitrogruppe(n), Aminogruppe(n) und/oder einem oder mehreren Halogenatom(en) - untereinander gleich oder verschieden - substituiert sein kann; bevorzugter Arylrest ist ein gegebenenfalls substituierter Phenylrest, wobei als Substituenten Halogen - wie Fluor, Chlor oder Brom - sowie Hydroxyl bevorzugt sind.

Alkoxy steht im allgemeinen für einen über ein Sauerstoffatom gebundenen geradkettigen oder verzweigten Kohlenwasserstoffrest mit 1 bis 8 Kohlenstoffatom(en). Bevorzugt ist ein Niederalkoxyrest mit 1 bis 3 Kohlenstoffatom(en). - Besonders bevorzugt ist die Methoxygruppe.

Wie gefunden wurde, zeichnen sich die Verbindungen der Formel I durch vielfältige Anwendungsmöglichkeiten auf therapeutischem Gebiet aus. Hervorzuheben sind solche Anwendungsmöglichkeiten, für welche die LTB₄rezeptorantagonistischen Eigenschaften eine Rolle spielen. Hier sind insbesondere zu nennen:
Arthritis, Asthma, chronische obstruktive Lungenerkrankungen, etwa chronische Bronchitis, Psoriasis, Colitis ulcerosa, durch nichtsteroidale Antiphlogistika induzierte Gastro- oder Enteropathie, cystische Fibrose, Alzheimer-Krankheit, Schock, Reperfusionsschäden/lschämien, Atherosklerose, Multiple Sklerose.

Auch lassen sich mit den neuen Verbindungen Krankheiten oder Zustände behandeln, bei denen die Passage von Zellen aus dem Blut über das vaskuläre Endothelium in das Gewebe von Bedeutung ist (etwa Metastasis) oder Krankheiten und Zustände, bei denen die Kombination des LTB₄ oder eines anderen Moleküls (beispielsweise 12-HETE) mit dem LTB₄-Rezeptor einen Einfluß auf die Zell-Proliferation hat (etwa chronische myelozytische Leukämie)

Die neuen Verbindungen können auch in Kombination mit anderen Wirkstoffen angewendet werden, etwa solchen. die für dieselben Indikationen Verwendung finden, oder z.B. mit Antiallergika, Sekretolytika, β₂-Adrenergika, inhalativ anwendbaren Steroiden, Antihistaminika und/oder PAF-Antagonisten. Die Verabreichung kann topisch, oral, transdermal, nasal, parenteral oder inhalativ erfolgen.

Zur pharmakolgischen und biochemischen Untersuchung der Wirkungsverhältnisse eigen sich Tests, wie sie beispielsweise in der WO 93/16036, S 15 bis 17 - auf die hier inhaltlich Bezug genommen wird - offenbart sind.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 10 und 500 mg, vorzugsweise zwischen 20 und 250 mg. Bei inhalativer Anwendung werden dem Patienten zwischen etwa 0,5 und 25, vorzugsweise zwischen etwa 2 und 20 mg Wirkstoff zugeführt.

Inhalationslösungen enthalten im allgemeinen zwischen etwa 0,5 und 5 % Wirkstoff. Die neuen Verbindungen können in üblichen Zubereitungen verabreicht werden, etwa als Tabletten, Dragées, Kapseln, Oblaten, Pulver, Granulate, Lösungen, Emulsionen, Sirupe, Inhalationsaerosole, Salben, Suppositorien.

### Formulierungsbeispiele

Die nachstehenden Beispiele zeigen einige Möglichkeiten für die Formulierung der Darreichungsformen :

### 1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.

### 2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

### 3. Inhalationspulver

Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 33 45 722, auf die hiermit inhaltlich Bezug genommen wird, inhaliert.

Die neuen Verbindungen können nach folgenden konventionellen Methoden hergestellt werden, die an sich aus dem Stand der Technik bekannt sind:
1. Reduktion eines Amidoxims der allgemeinen Formel 2 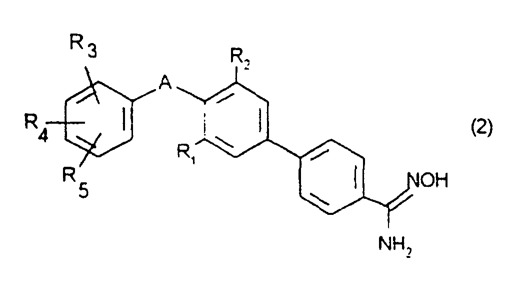 worin R₁ bis R₅ und A die oben angegebene Bedeutung haben.
   Für die Reduktion des Amidoxims der allgemeinen Formel 2 eignet sich die katalytische Hydrierung, insbesondere mit Raney-Nickel in einem niederen Alkohol, z.B. Methanol.
   Zweckmäßigerweise wird das Amidoxim der allgemeinen Formel 2 unter Zugabe der berechneten Menge derjenigen Säure, deren Salz als Endprodukt gewünscht wird, in Methanol gelöst und bei Raumtemperatur unter leichtem Druck, z.B. bei 5 bar, bis zur beendeten Wasserstoffaufnahme hydriert.
2. Umsetzung von Iminoestern der allgemeinen Formel 3 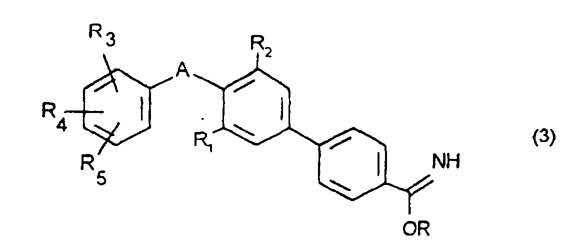 in der R₁ bis R₅ und A die obige Bedeutung haben und R bevorzugt für einen niederen Alkylrest steht, mit Ammoniak.
   Die Umsetzung erfolgt zweckmäßig in einem organischen Lösungsmittel bei Temperaturen zwischen etwa 0°C und der Siedetemperatur des Reaktionsgemischs, vorzugsweise zwischen Raumtemperatur und etwa 100°C bzw. der Siedetemperatur, soweit diese niedriger ist. Geeignete Lösungsmittel sind polare Lösungsmittel wie Methanol, Ethanol, Propanole.
3. Umsetzung eines Phenols der allgemeinen Formel 4 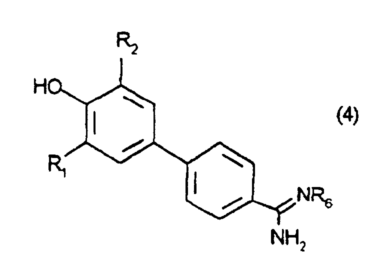 worin R₁, R₂, R₆ die oben angegebene Bedeutung haben, mit einer Verbindung der Formel 5 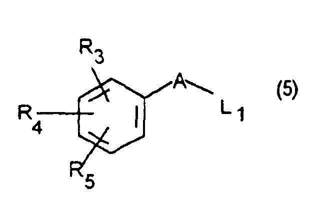 worin A, R₃, R₄, R₅ die obige Bedeutung haben und L₁ eine nucleofuge Abgangsgruppe wie ein Halogenatom oder einen Sulfonsäurerest darstellt.
   Die Umsetzung erfolgt in aprotischen Lösungsmitteln wie Dimthylsulfoxid, Dimethylformamid, Acetonitril oder Alkoholen wie Methanol, Ethanol oder Propanol unter Zusatz einer Base (vorzugsweise eines Alkali- oder Eralkalimetallcarbonats, -hydroxids oder -hydride) bei Temperaturen zwischen etwa 0 und 140°C bzw. der Siedetemperatur des Reaktionsgemischs.
4. Umsetzung eines Amidins der allgemeinen Formel 6 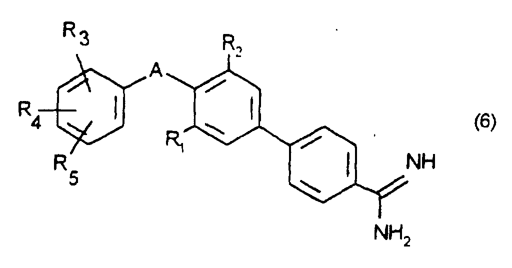 in der R₁ bis R₅ und A die oben angegebene Bedeutung haben, mit einer Verbindung der Formel 7

   L₂-R₆' (7)

   in der R₆' dieselbe Bedeutung wie R₅ hat, ausgenommen H, und L₂ eine nucteofuge Austrittsgruppe wie ein Halogenatom (etwa Cl, Br) oder Acyloxy bedeutet.
   Die Umsetzung wird zweckmäßigerweise in einem Lösungsmittel wie Tetrahydrofuran, Methylenchlorid, Chloroform oder Dimethylformamid, vorzugsweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methylmorpholin oder Pyridin, welche gleichzeitig als Lösungsmittel dienen können, bei Temperaturen zwischen -30 und 100°C, vorzugsweise jedoch bei Temperaturen zwischen -10 und 80°C, durchgeführt.

Die erfindungsgemäßen Verbindungen sind ausgehend von aus dem Stand der Technik bekannten Verbindungen u.a. nach den in den folgenden Beispielen beschriebenen Verfahren herstellbar. Verschiedenartige, andere Ausgestaltungen der Verfahren werden für den Fachmann aus der vorliegenden Beschreibung ersichtlich. Es wird jedoch ausdrücklich darauf hingewiesen, daß diese Beispiele und die diesen zugeordnete Beschreibung lediglich zum Zweck der Erläuterung vorgesehen und nicht als Einschränkung der Erfindung anzusehen sind. 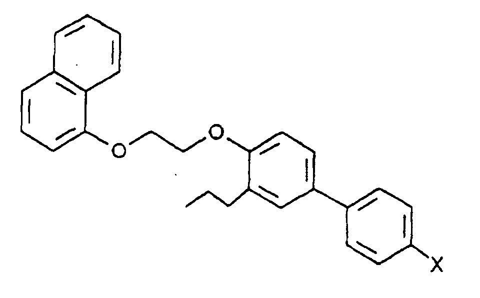 Amidoxim: X = C(=NOH)NH₂
5,3 g des Nitrils der obigen Formel (X = CN) wird zusammen mit 98 ml Ethanol, 3,65 g Hydroxylamin x HCI, 2,9 g Na₂CO₃ und 21 ml Wasser 5 Stunden am Rückfluß gekocht. Danach wird bis zur Trockne abdestilliert und der Rückstand mit Wasser verrührt und
abgesaugt. 5,7 g wird in 25 ml Acetonitril aufgeschlämmt und mit 0,85 ml Methansulfonsäure versetzt, erwärmt und wieder abgekühlt.
Nach dem Absaugen erhält man 6,4 g des Methansulfonats als weiße Kristalle.
Amidin: X = C(=NH)-NH₂
6,4 g des Amidoxims [X = C(=NOH)-NH₂] als Methansulfonat wird in 100 ml Methanol gelöst und mit Raney-Nickel als Katalysator unter Normalbedingungen bis zur 100 %igen Wasser-stoffaufnahme hydriert. Nach Absaugen wird eingeengt und der Rückstand aus Methanol umkristallisiert. Ausbeute: 3,8 g der Amidinverbindung als Methansulfonat. Fp. 228 - 30°C.
Ethoxycarbonylamidin: X = C(NCOOC₂H₅)-NH₂
2,6 g des Amidinmethansulfonats wird mit 1,6 ml Triethylamin in 50 ml Essigester suspen-diert und dazu läßt man 0,6 ml Chlorameisensäureethylester in 5,5 ml Essigester bei Raum-temperatur in etwa 15 Minuten zutropfen. Das Reaktionsgemisch wird dann dreimal mit Wasser gewaschen, mit Na₂SO₄ getrocknet und eingedampft. Nach Umkristallisieren aus Acetonitril erhält man die Ethoxycarbonylamidinverbindung vom Fp. 142 - 144°C.

Gemäß dieser Vorschrift werden u.a. folgende Verbindungen erhalten:

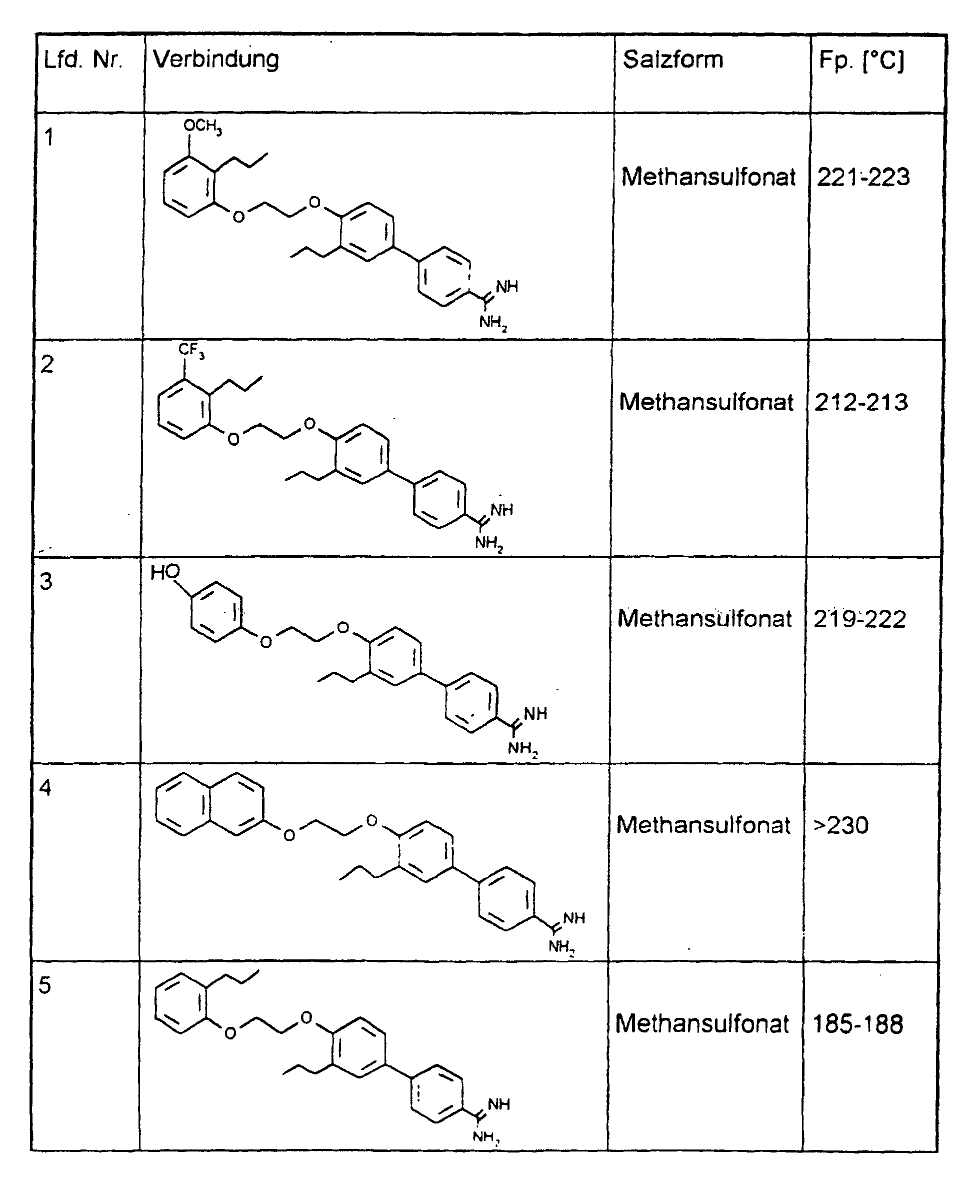

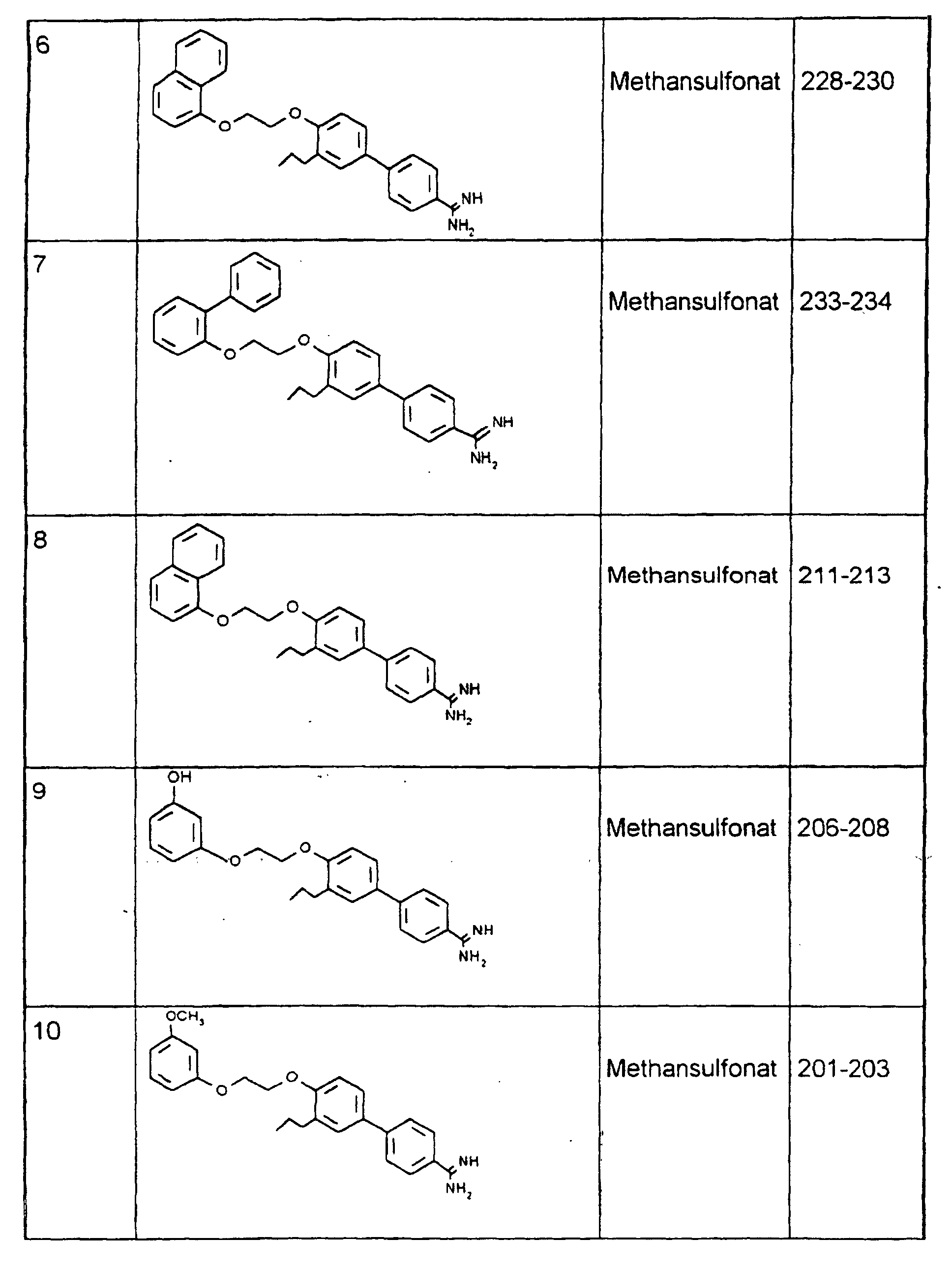

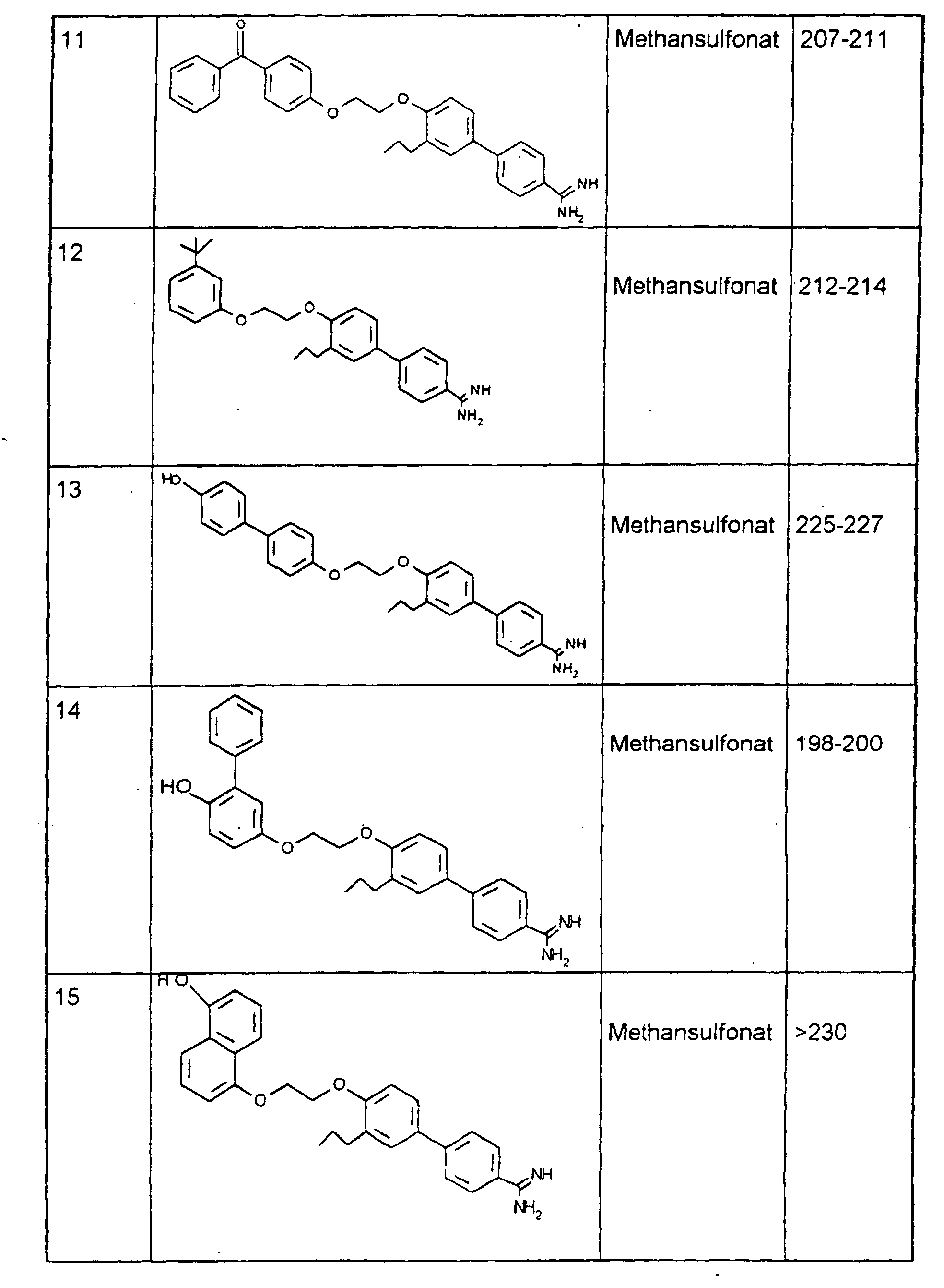

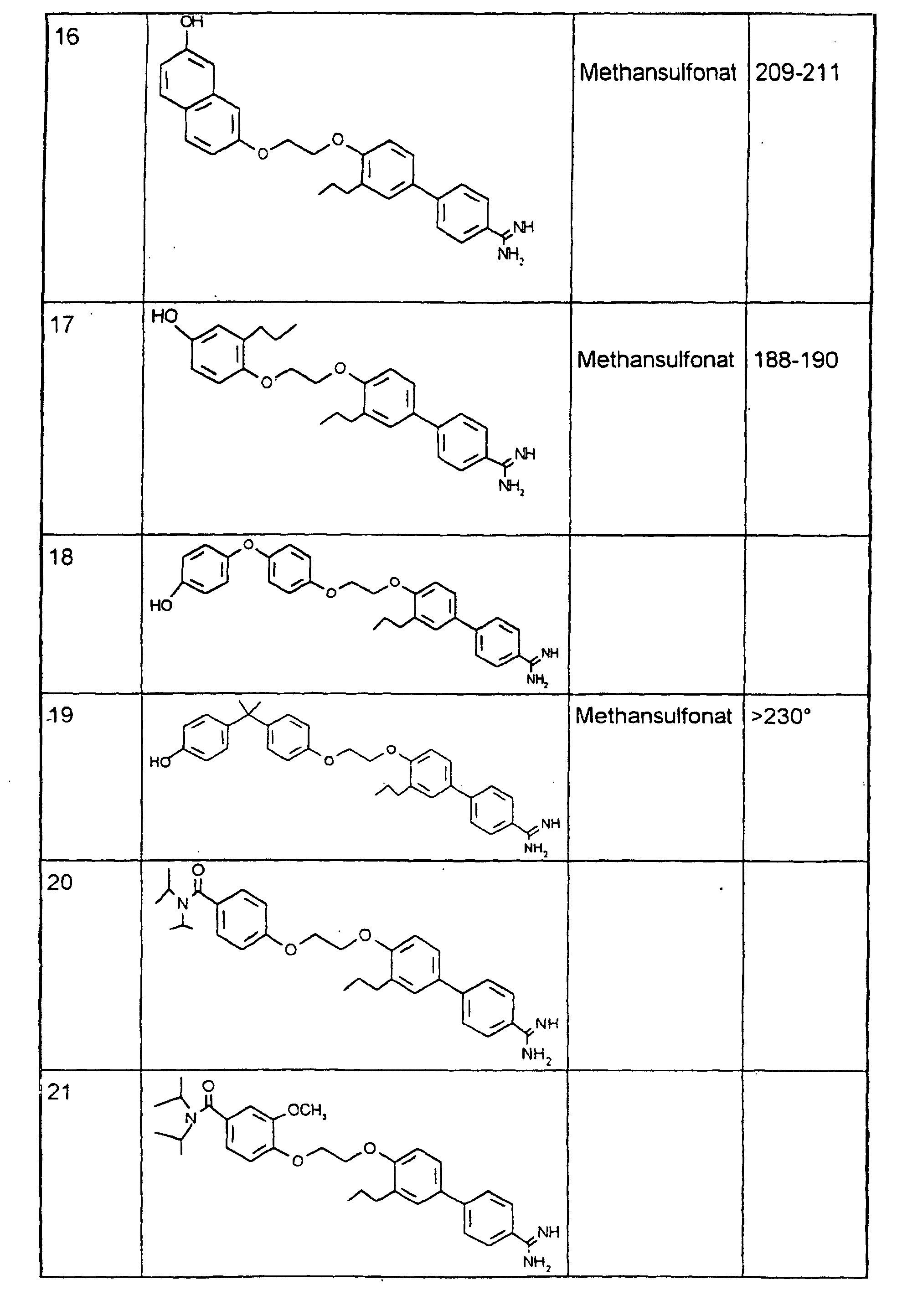

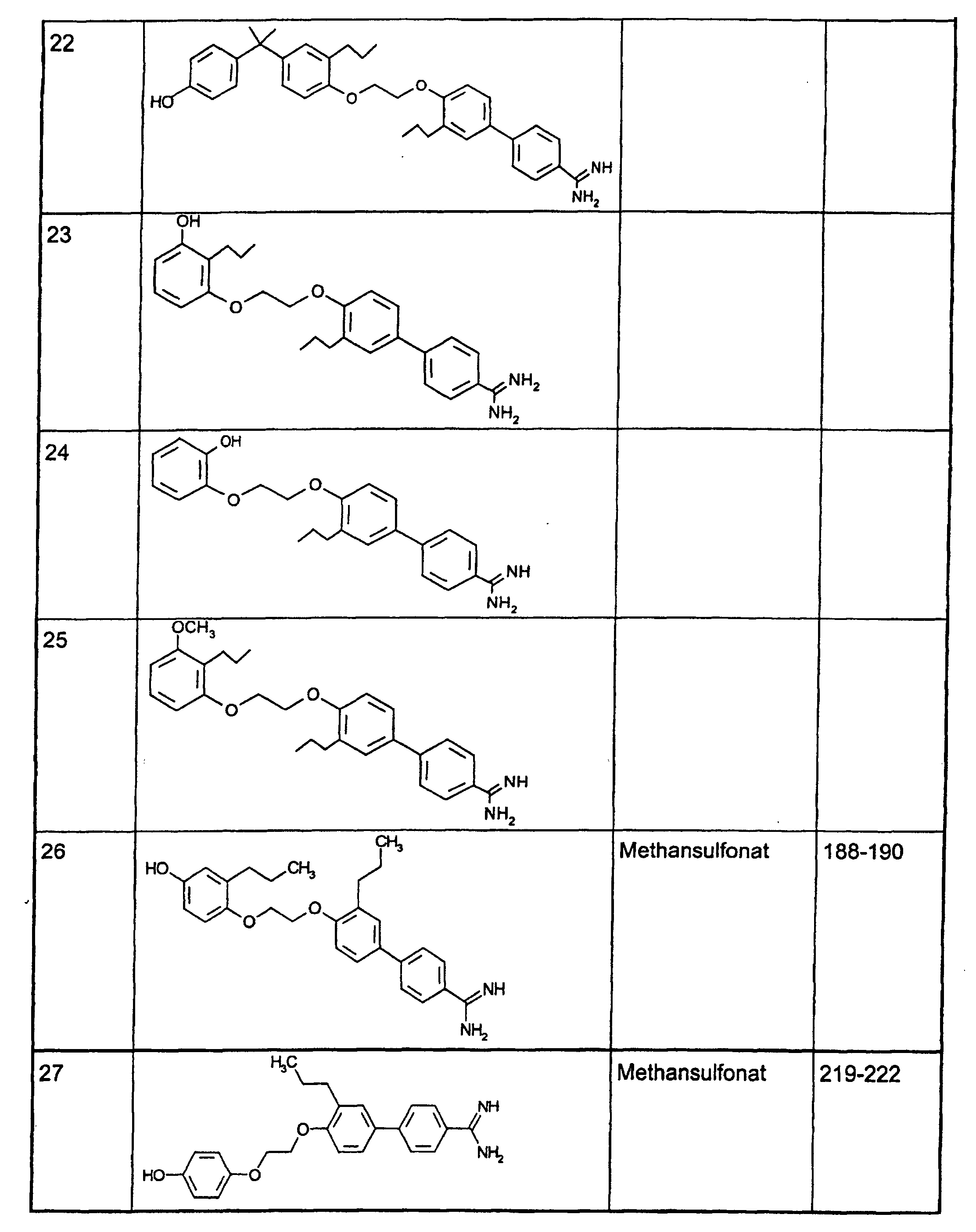

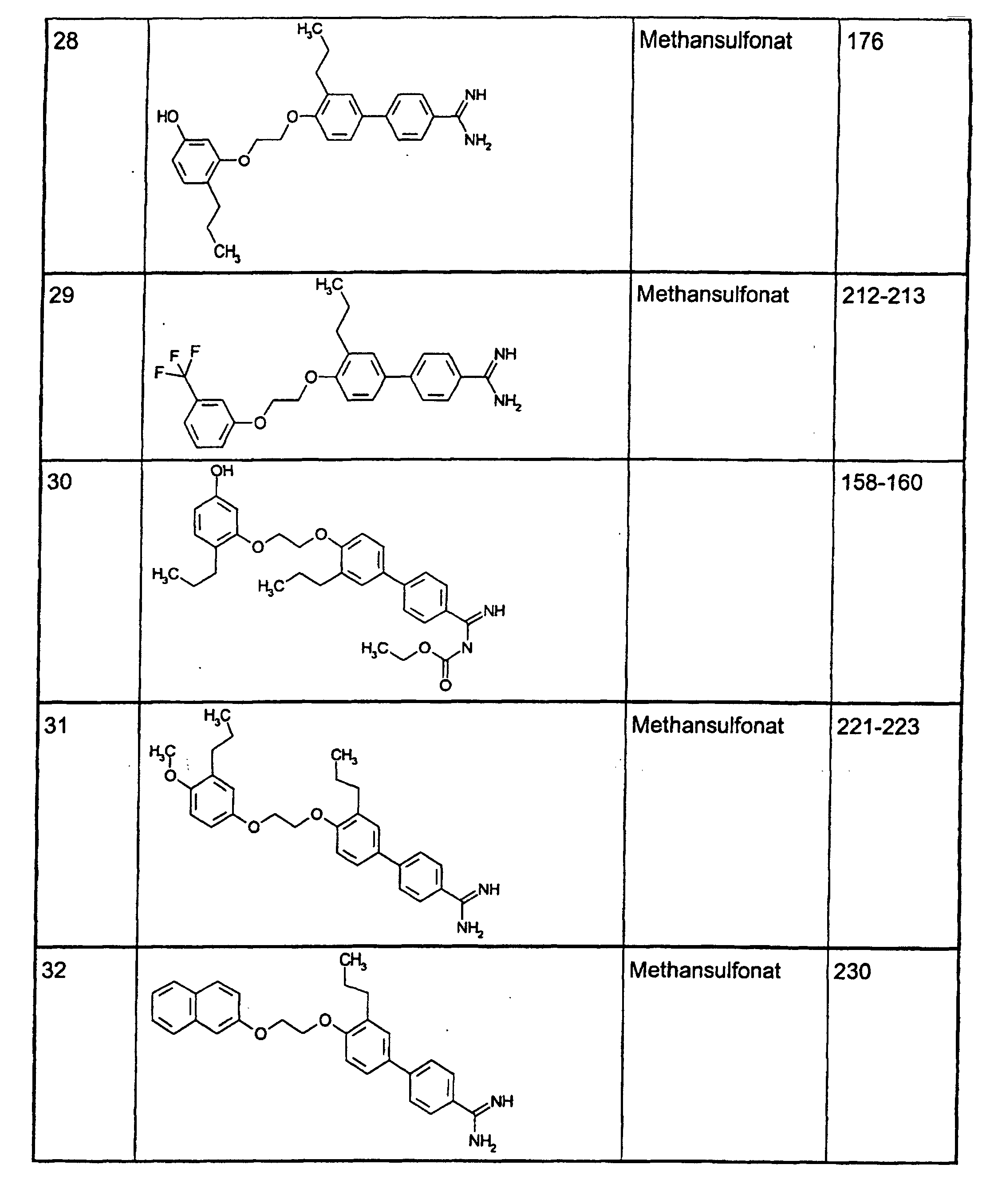

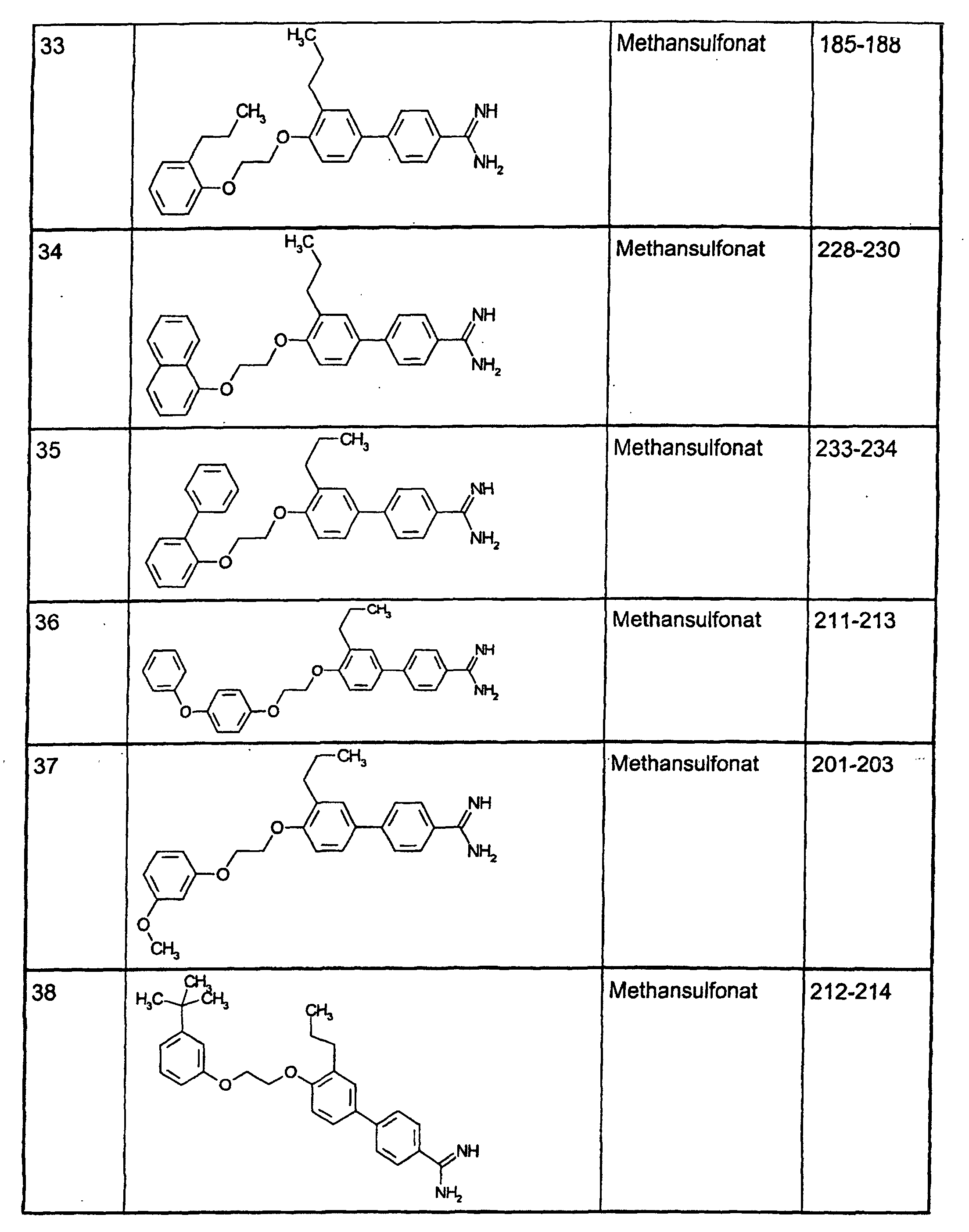

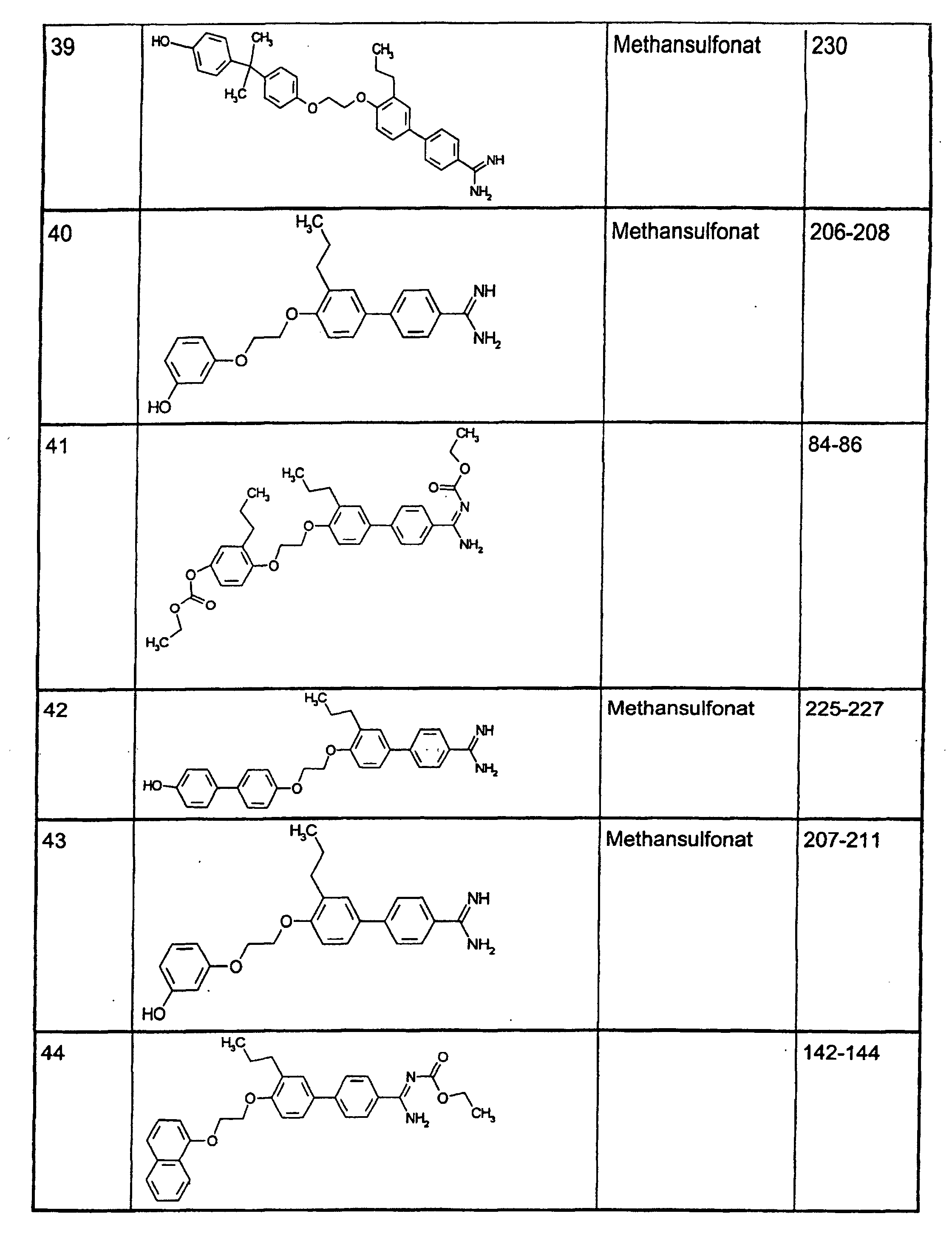

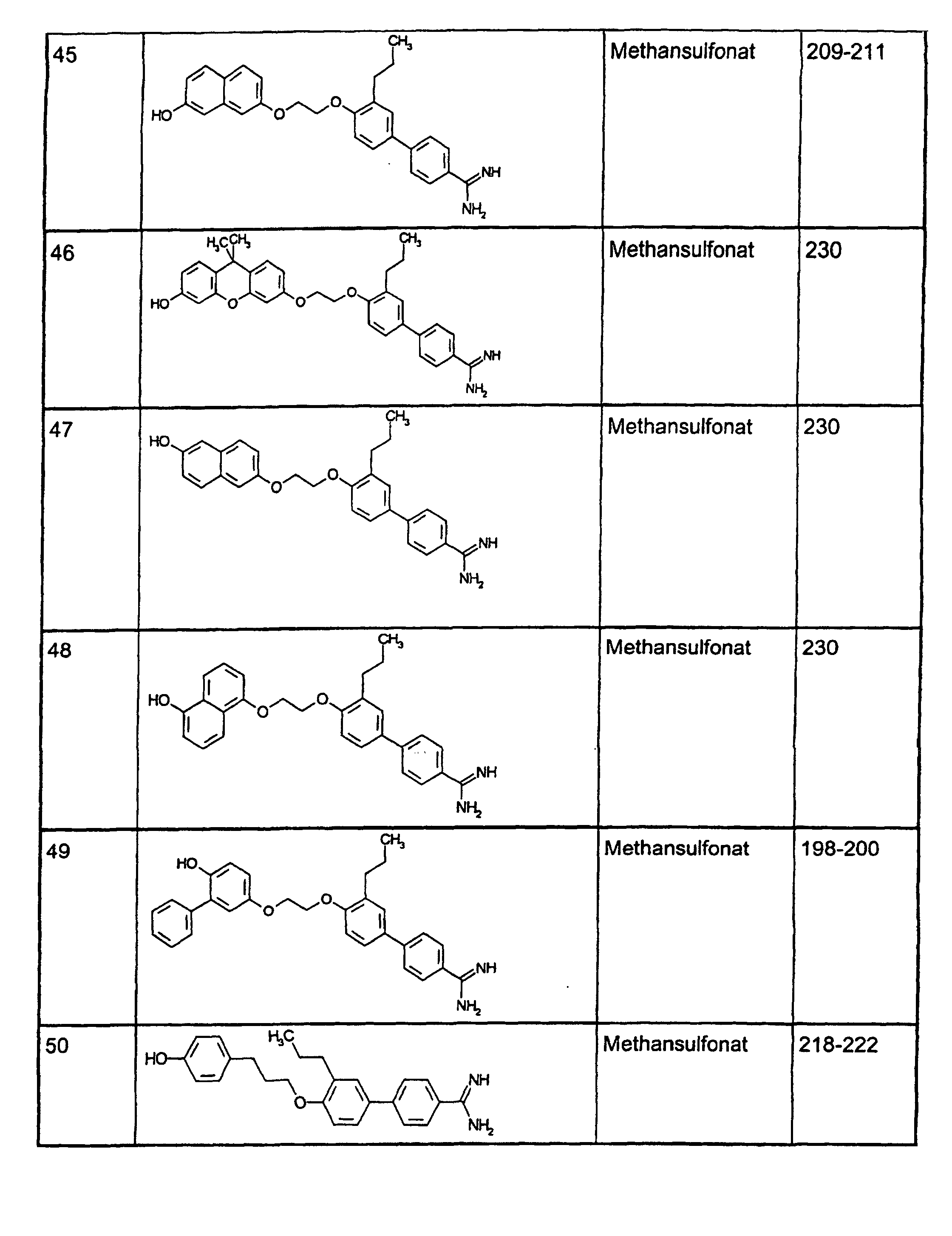

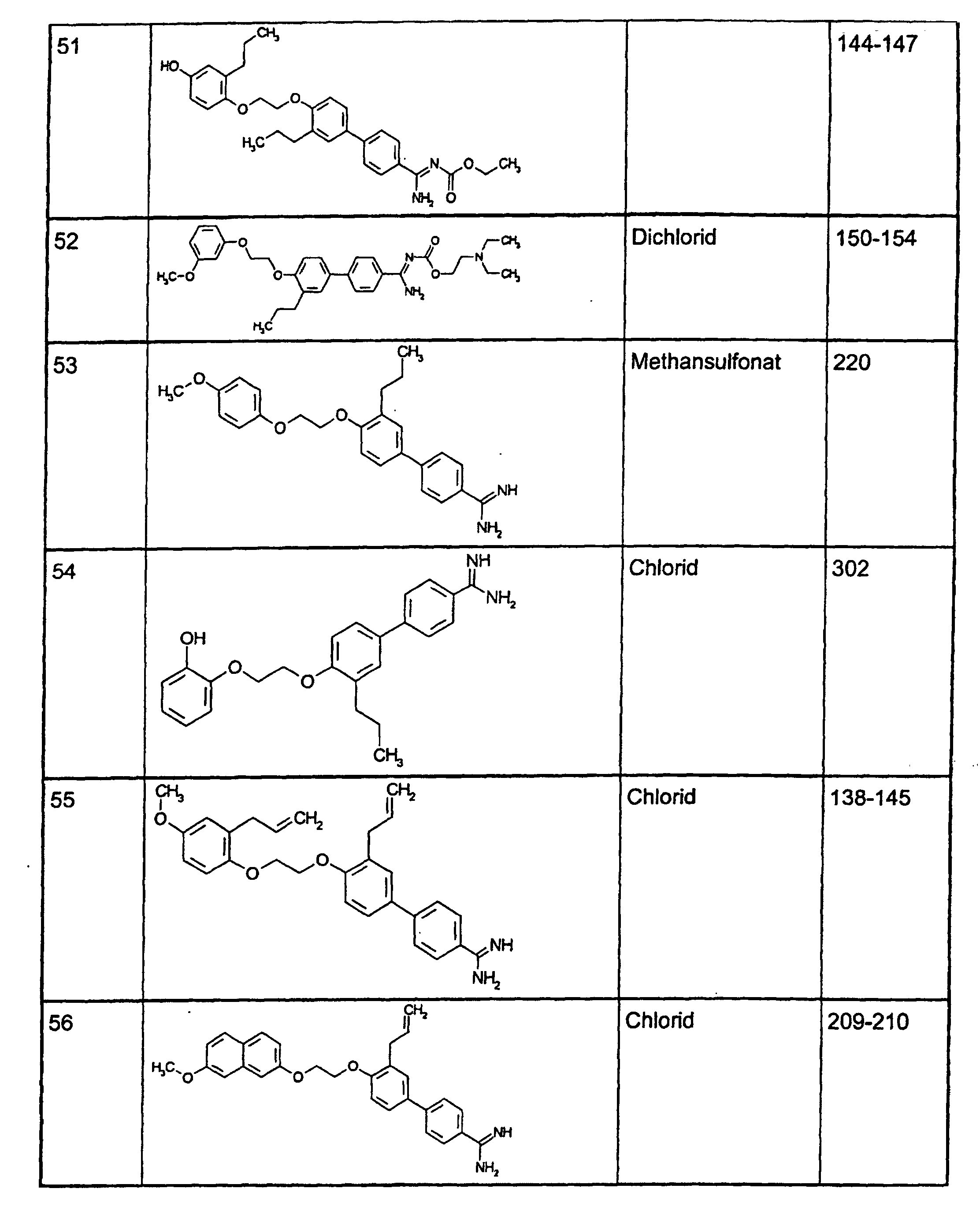

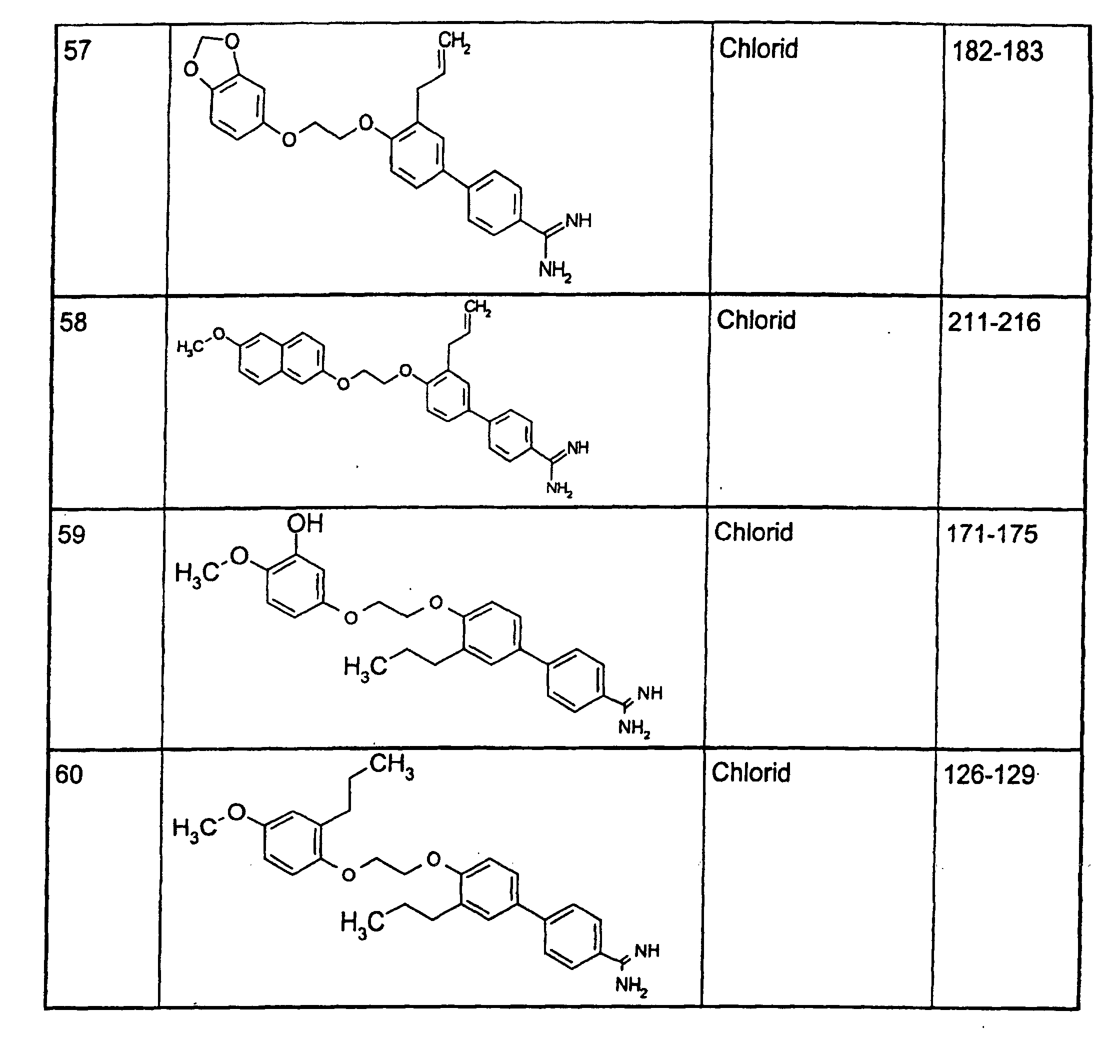

## Patentansprüche

1. Benzamidinderivate der allgemeinen Formel 1 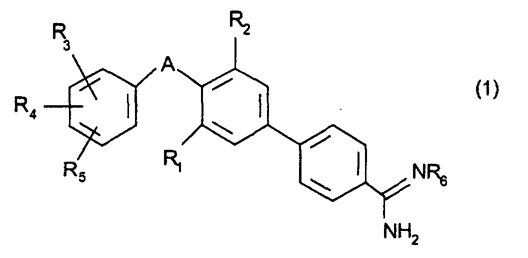 worin
A -OC₂-C₄-AlkylenO- oder -C₁-C₃-AlkylenO-
R₁ C₁-C₆-Alkyl, verzweigt oder unverzweigt, C₃-C₆-Alkenyl, verzweigt oder unverzweigt, vorzugsweise Allyl oder F, Cl, Br, I;
R₂ Wasserstoff, C₁-C₆-Alkyl, verzweigt oder unverzweigt, C₃-C₆-Alkenyl, verzweigt oder unverzweigt, vorzugsweise Allyl oder F, Cl, Br, I;
R₃ und R₄, die gleich oder verschieden sein können, unabhängig voneinander Wasserstoff, C₁-C₆-Alkyl, verzweigt oder unverzweigt, C₃-C₆-Alkenyl, verzweigt oder unverzweigt, vorzugsweise Allyl, C₁-C₆-Alkoxy, (C₁-C₄-Alkyl)OC(O)O-, OH, CF₃ oder zusammen ein ankondensiertes einoder zweikerniges Ringsystem bilden können, das vollständig oder teilweise durchkonjugiert sein kann und gegebenenfalls ein oder zwei Hetertoatome aus der Gruppe Sauerstoff, Schwefel oder Stickstoff aufweisen kann, die gleich oder verschieden sein können, das gegebenenfalls durch OH, C₁-C₄-Alkoxy, C₁-C₄-Alkyl substituiert sein kann;
R₅ Wasserstoff, Aryl, bevorzugt Phenyl, -OPhenyl, -CR₇R₈Phenyl, -COPhenyl, -SO₂-Phenyl, -CH(OH)Phenyl, wobei der Phenylring durch OH, C₁-C₄-Alkoxy substituiert sein kann, oder -C(O)NR₉R₁₀;
R₆ Wasserstoff, C₁-C₆-Alkoxycarbonyl, (C₁-C₅-Alkyl)-carbonyl oder -C(O)-O-(C₁-C₆-Alkylen)-NR₁₁R₁₂;
R₇ und R₈, die gleich oder verschieden sein können, Wasserstoff, C₁-C₈-Alkyl, verzweigt oder unverzweigt;
R₉ und R₁₀, die gleich oder verschieden sein können, unabhängig voneinander Wasserstoff, C₁-C₈-Alkyl, verzweigt oder unverzweigt;
R₁₁ und R₁₂, die gleich oder verschieden sein können, unabhängig voneinander Wasserstoff, C₁-C₈-Alkyl, verzweigt oder unverzweigt;
bedeuten können,
gegebenenfalls in Form der einzelnen optischen Isomeren, Mischungen der einzelnen Enantiomeren oder Racemate sowie in Form der freien Basen oder der entsprechenden Säureadditionssalze mit pharmakologisch unbedenklichen Säuren.

2. Verbindungen der allgemeinen Formel 1 nach Anspruch 1, in der
A -OCH₂CH₂O-, -CH₂O-, -CH₂CH₂CH₂O-;
R₁ C₁-C₅-Alkyl, verzweigt oder unverzweigt, Allyl;
R₂ Wasserstoff;
R₃ Wasserstof, C₁-C₆-Alkyl, OCH₃, C₂H₅OC(O)O-, OH, Cl, F, CF₃;
R₄ Wasserstoff. -OCH₃, OH;
R₃ und R₄ zusammen einen ankondensierten Benzolring, ein 3,4-Dihydrocumarinsystem oder ein 1,3-Dioxolansystem; der durch OH, C₁-C₃-Alkyl, OCH₃ substituiert sein kann;
R₅ Wasserstoff, Phenyl, OPhenyl, -CR₇R₈Phenyl, wobei der Phenylring durch OH, OCH₃ substituiert sein kann, oder -C(O)NR₉R₁₀;
R₆ Wasserstoff, C₁-C₄-Alkoxycarbonyl oder -C(O)-O-(CH₂)₂-N(C₂H₅)₂;
R₇ und R₈, die gleich oder verschieden sein können, unabhhängig voneinander Wasserstoff, CH₃ oder CF_{3;}
R₉ und R₁₀, die gleich oder verschieden sein können, Wasserstoff, C₁-C₈-Alkyl, verzweigt oder unverzweigt;
bedeuten können,
gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren.

3. Verfahren zur Herstellung von Verbindungen der allgemeinen Formel 1, **dadurch gekennzeichnet, daß** man ein Amidoxim der allgemeinen Formel 2 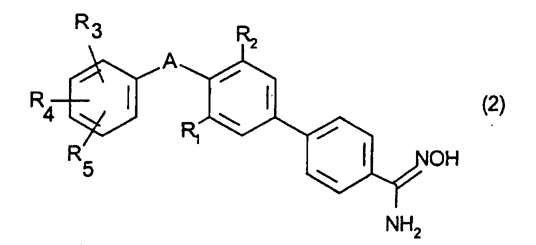 worin R₁ bis R₅ und A die in Anspruch 1 angegebene Bedeutung haben, auf an sich bekannte Weise reduziert.

4. Verfahren nach Anspruch 3, **dadurch gekennzeichnet, daß** man die Reduktion auf kataytischem Wege, bevorzugt in Gegenwart von Raney-Nickel, durchführt.

5. Verfahren nach Anspruch 3 oder 4, **dadurch gekennzeichnet, daß** die katalytische Reduktion des Amidoxims in einem niederen Alkohol, bevorzugt in Methanol, durchgeführt wird.

6. Verfahren zur Herstellung von Verbindungen der allgemeinen Formel 1, **dadurch gekennzeichnet, daß** man 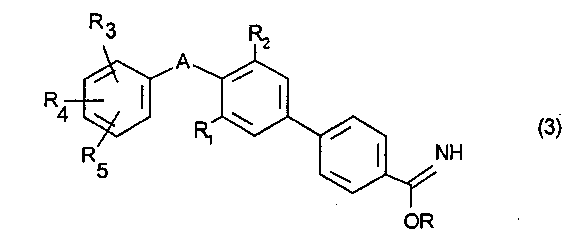 in der R₁ bis R₅ und A worin R₁ bis R₅ und A die in Anspruch 1 angegebene Bedeutung haben, mit Ammoniak umsetzt.

7. Verfahren nach Anspruch 6, **dadurch gekennzeichnet, daß** die Umsetzung in einem organischen Lösungsmittel bei Temperaturen zwischen etwa 0°C und der Siedetemperatur des Reaktionsgemischs, bevorzugt in einem Temperaturintervall zwischen Raumtemperatur und etwa 100°C bzw. der Siedetemperatur, soweit diese niedriger ist, erfolgt.

8. Verfahren nach Anspruch 6 oder 7, **dadurch gekennzeichnet, daß** die Umsetzung in einem polaren Lösungsmittel, bevorzugt Methanol, Ethanol oder in einem Propanol, erfolgt.

9. Verfahren zur Herstellung von Verbindungen der allgemeinen Formel 1, **dadurch gekennzeichnet, daß** man ein Phenol der allgemeinen Formel 4 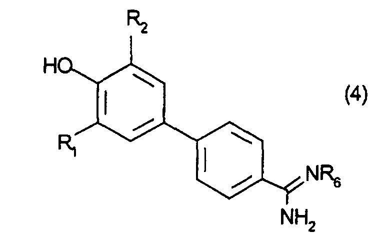 worin R₁, R₂, R₆ die in Anspruch 1 angegebene Bedeutung haben, mit einer Verbindung der Formel 5 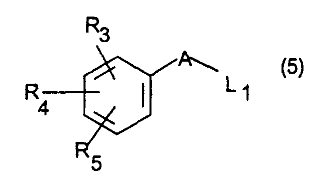 worin A, R₃, R₄, R₅ die in Anspruch 1 angegebene Bedeutung haben und L₁ eine nucleofuge Abgangsgruppe, bevorzugt ein Halogenatom oder einen Sulfonsäurerest, bedeutet, in einem aprotischen Lösungsmittel umsetzt.

10. Verfahren nach Anspruch 9, **dadurch gekennzeichnet, daß** das Lösungsmittel Dimethylsulfoxid, Dimethylformamid, Acetonitril oder ein Alkohol, bevorzugt Methanol, Ethanol oder ein Propanol ist.

11. Verfahren nach Anspruch 9 oder 10, **dadurch gekennzeichnet, daß** die Umsetzung in Gegenwart einer Base, bevorzugt in Gegenwart eines Alkalimetall- oder Erdalkalimetallcarbonats, Metallhydroxids oder Metallhydrids, durchgeführt wird.

12. Verfahren nach einem der Ansprüche 9 bis 11, **dadurch gekennzeichnet, daß** die Umsetzung bei einer Temperatur in einem Intervall zwischen 0 °C und dem Siedepunkt der Reaktionsmischung und bevorzugt in einem Temperaturintervall von 0 bis 140°C durchgeführt wird.

13. Verfahren zur Herstellung von Verbindungen der allgemeinen Formel 1, **dadurch gekennzeichnet, daß** man ein Amidin der allgemeinen Formel 6 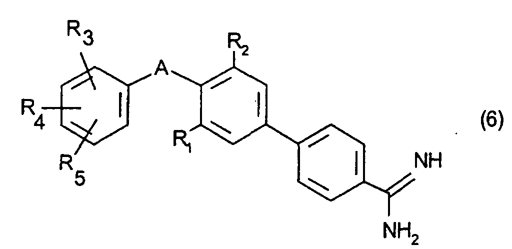 in der R₁ bis R₅ und A die in Anspruch1 angegebene Bedeutung haben, mit einer Verbindung der allgemeinen Formel 7
L₂ - R₆' (7)
in der
R₆' C₁-C₆-Alkoxycarbonyl oder C₁-C₆-Acyl, und
L₂ eine nucleofuge Austrittsgruppe, bevorzugt Chlor, Brom oder Acyloxy, bedeuten, umsetzt.

14. Verfahren nach Anspruch 13, **dadruch gekennzeichnet, daß** dieUmsetzung in einem polaren Lösungsmittel erfolgt.

15. Verfahren nach Anspruch 14, **dadurch gekennzeichnet, daß** dasLösungsmittel Tetrahydrofuran, Methylenchlorid, Chloroform oder Dimethylformamid ist.

16. Verfahren nach Anspruch 14 oder 15, **dadurch gekennzeichnet, daß** die Umsetzung in Gegenwart einer anorganischen Base, vorzugsweise Natriumcarbonat, Kaliumcarbonat oder Natronlauge, oder in Gegenwart einer tertiären organischen Base, vorzugsweise Triethylamin, N-Ethyl-diisopropylamin, N-Methylmorpholin oder Pyridin erfolgt.

17. Verfahren nach einem der Ansprüche 14 bis 16, **dadurch gekennzeichnet, daß** die Umsetzung in einem Temperaturintervall zwischen -30 und 100°C, vorzugsweise in einem Temperaturintervall zwischen -10 und 80°C, durchgeführt wird.

18. Pharmazeutische Zubereitung, **gekennzeichnet durch** einen Gehalt an einer Verbindung nach einem der Ansprüche 1 bis 2 und deren Säureadditionssalze neben üblichen Hilfs- und Trägerstoffen.

19. Verbindung der Formel (1) nach einem der Ansprüche 1 bis 2 zur Verwendung als Arzneimittel.

20. Verwendung von Verbindungen der Formel (1) nach einem der Ansprüche 1 bis 2 zur Herstellung eines Arzneimittels mit LTB₄antagonistischer Wirkung.

21. Verwendung von Verbindungen der allgemeinen Formel 1, deren Stereoisomere sowie deren Säureadditionssalze zur Herstellung eines Medikaments zur therapeutischen Behandlung von Arthritis, Asthma, chronischer obstruktiver Lungenerkrankung wie chronischer Bronchitis, Psoriasis, Colitis ulcerosa, durch nichtsteroidale Antiphlogistika induzierter Gastro- oder Enteropathie, cystischer Fibrose, Alzheimer-Krankheit, Schock, Reperfusionsschäden/Ischämien, Atheriosklerose, multipler Sklerose.

## Claims

1. Benzamidine derivatives of general formula 1 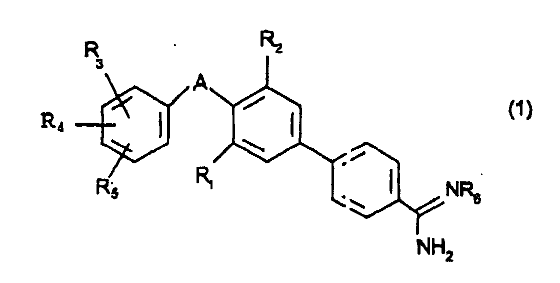 wherein
A denotes -O-C₂-₄-alkylene-C- or -C₁-₃-alkylene-O-
R₁ denotes branched or unbranched C₁₋₆-alkyl, branched or unbranched C₃₋₆-alkenyl, preferably allyl, or F, Cl, Br, I;
R₂ denotes hydrogen, branched or unbranched C₁₋₆-alkyl, branched or unbranched C₃-₆-alkenyl, preferably allyl, or F, Cl, Br, I;
R₃ and R₄, which may be identical or different, independently of one another denote hydrogen, branched or unbranched C₁₋₆-alkyl, branched or unbranched C₃₋₆-alkenyl, preferably allyl, C₁₋₆-alkoxy, (C₁₋₄-alkyl)OC(O)O, OH or CF₃ or together denote a fused-on mono- or dinuclear ring system which may be wholly or partially conjugated and may optionally contain one or more heteroatoms selected from the group comprising oxygen, sulphur or nitrogen, which may be identical or different, which may optionally be substituted by OH, C₁-₄-alkoxy or C₁-C₄-alkyl;
R₅ denotes hydrogen, aryl, preferably phenyl, -O-phenyl, -CR₇R₈-phenyl, -C(O)phenyl, -SO₂phenyl, -CH(OH)phenyl, wherein the phenyl ring may be substituted by OH or -C₁-₄-alkoxy, or it denotes -C(O)NR₉R₁₀;
R₆ denotes hydrogen, C₁-₆-alkoxycarbonyl or (C₁-₅-alkyl)carbonyl or -C(O)-O- (C₁-₆-alkylene)-NR₁₁R₁₂;
R₇ and R₈, which may be identical or different, denote hydrogen or branched or unbranched C₁-₈-alkyl;
R₉ and R₁₀, which may be identical or different, independently of one another denote hydrogen or branched or unbranched C₁₋₈-alkyl;
R₁₁ and R₁₂, which may be identical or different, independently of one another denote hydrogen or branched or unbranched C₁-₈-alkyl;
optionally in the form of the individual optical isomers, mixtures of the individual enantiomers or racemates and in the form of the free bases or the corresponding acid addition salts with pharmacologically acceptable acids.

2. Compounds of general formula 1 according to claim 1, wherein
A denotes -OCH₂CH₂O-, -CH₂O-, -CH₂CH₂CH₂O-;
R₁ denotes branched or unbranched C₁₋₅-alkyl or allyl;
R₂ denotes hydrogen;
R₃ denotes hydrogen, C₁-₆-alkyl, OCH₃, C₂H₅OC(O)O, OH, Cl, F, CF₃;
R₄ denotes hydrogen, -OCH₃, OH;
R₃ and R₄ together denote a fueled-on benzene ring, a 3,4-dihydrocumarin system or a 1,3-dioxolane system which may be substituted by OH, C₁-C₃-alkyl or OCH₃;
R₅ denotes hydrogen, phenyl, O-phenyl, -CR₇R₈-phenyl, wherein the phenyl ring may be substituted by OH or OCH₃, or it denotes -C(O)NR₉R₁₀;
R₆ denotes hydrogen or C₁-₄-alkoxycarbonyl or -C(O)-O-(CH₂)₂-N(C₂H₅)₂;
R₇ and R₈, which may be identical or different, ind dependently of one another denote hydrogen, CH₃ or CF_{3;}
R₉ and R₁₀, which may be identical or different, denote hydrogen or branched or unbranched C₁-C₈-alkyl,
optionally in the form of the individual optical isomers, mixtures of the individual enantiomers or racemates and in the form of the free bases or the corresponding acid addition salts with pharmacologically acceptable acids.

3. Process for preparing compounds of general formula 1, **characterised in that** an amidoxime of general formula 2 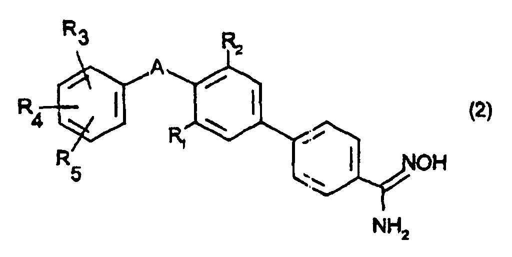 wherein R₁ to R₅ and A are defined as in claim 1, is reduced in a manner known *per se*.

4. Process according to claim 3, **characterised in that** the reduction is carried out catalytically, preferably in the presence of Raney nickel.

5. Process according to claim 3 or 4, **characterised in that** the catalytic reduction of the amidoxime is carried out in a lower alcohol, preferably in methanol.

6. Process for preparing compounds of general formula 1, **characterised in that** 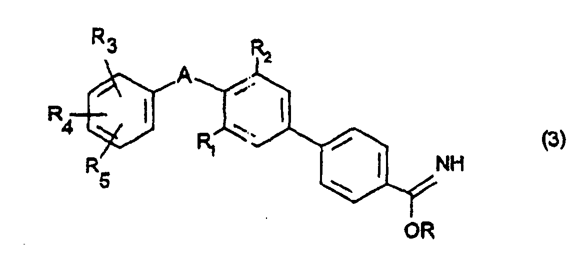 wherein R₁ to R₅ and A wherein R₁ to R₅ and A [*sic*] are defined as in claim 1, is reacted with ammonia.

7. Process according to claim 6, **characterised in that** the reaction is carried out in an organic solvent at temperatures between about 0°C and the boiling temperature of the reaction mixture, preferably in a temperature range of between ambient temperature and about 100°C or at the boiling temperature, if this is lower.

8. Process according to claim 6 or 7, **characterised in that** the reaction is carried out in a polar solvent, preferably methanol, ethanol or a propanol.

9. Process for preparing compounds of general formula 1, **characterised in that** a phenol of general formula 4 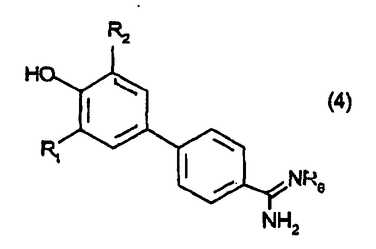 wherein R₁, R₂ and R₆ are defined as in claim 1, is reacted with a compound of formula 5 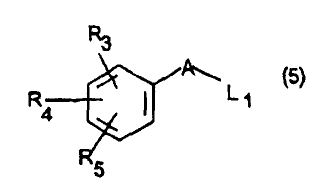 wherein A, R₃, R₄ and R₅ are defined as in claim 1 and L₁ denotes a nucleofugic leaving group, preferably a halogen atom or a sulphonic acid group, in an aprotic solvent.

10. Process according to claim 9, **characterised in that** the solvent is dimethylsulphoxide, dimethylformamide, acetonitrile or an alcohol, preferably methanol, ethanol ox a propanol.

11. Process according to claim 9 or 10, **characterised in that** the reaction is carried out in the presence of a base, preferably in the presence of an alkali metal or alkaline earth metal carbonate, metal hydroxide or metal hydride.

12. Process according to one of claims 9 to 11, **characterised in that** the reaction is carried out at a temperature in the range between 0°C and the boiling point of the reaction mixture and preferably in the temperature range from 0 to 140°C.

13. Process for preparing compounds of general formula 1, **characterised in that** an amidine of general formula 6 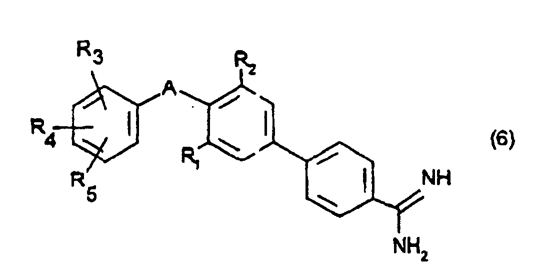 wherein R₁ to R₅ and A are defined as in claim 1, is reacted with a compound of general formula 7
L₂ - R₆' (7)
wherein R₆' denotes C₁-C₆-alkoxycarbonyl or C₁-C₆-acyl and L₂ denotes a nucleofugic leaving group, preferably chlorine, bromine or acyloxy.

14. Process according to claim 13, **characterised in that** the reaction is carried out in a polar solvent.

15. Process according to claim 14, **characterised in that** the solvent is tetrahydrofuran, methylene chloride, chloroform or dimethylformamide.

16. Process according to claim 14 or 15, **characterised in that** the reaction is carried out in the presence of an inorganic base, preferably sodium carbonate, potassium carbonate or sodium hydroxide solution, or in the presence of a tertiary organic base, preferably triethylamine, N-ethyl-diisopropylamine, N-methylmorpholine or pyridine.

17. Process according to one of claims 14 to 16, **characterised in that** the reaction is carried out in a temperature range of between -30 and 100°C, preferably in a temperature range of between -10 and 80°C.

18. Pharmaceutical preparation, **characterised in that** it contains a compound according to one of claims 1 and 2 and the acid addition salts thereof together with conventional excipients and/or carriers.

19. The compound of formula (1) according to one of claims 1 and 2 for use as a pharmaceutical composition.

20. Use of a compound of formula (1) ) according to one of claims 1 and 2 for preparing a pharmaceutical composition with an LTB₄-antagonistic activity.

21. Use of compounds of general formula 1, the stereoisomers and the acid addition salts thereof for the preparation of a medicament for the therapeutic treatment of arthritis, asthma, chronic obstructive lung diseases such as chronic bronchitis, psoriasis, ulcerative colitis, gastropathy or enteropathy induced by nonsteroidal antiinflammatories, cystic fibrosis, Alzheimer's disease, shock, reperfusion damage/ischaemia, atherosclerosis and multiple sclerosis.

## Revendications

1. Dérivés de benzamidine de formule générale 1 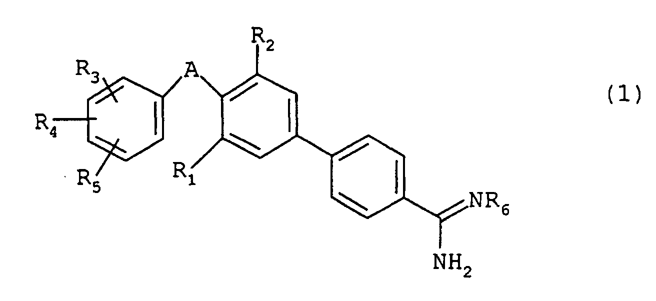 où
A peut représenter -O-alkylène en C₂-C₄-O- ou alkylène en C₁-C₃-O-
R₁ peut représenter alkyle en C₁-C₆, ramifié ou non ramifié, alcényle en C₃-C₆, ramifié ou non ramifié, de préférence allyle ou F, Cl, Br, I ;
R₂ peut représenter l'hydrogène, alkyle en C₁-C₆, ramifié ou non ramifié, alcényle en C₃-C₆, ramifié ou non ramifié, de préférence allyle ou F, Cl, Br, I ;
R₃ et R₄, qui peuvent être identiques ou différents, peuvent représenter indépendamment l'un de l'autre l'hydrogène, alkyle en C₁-C₆, ramifié ou non ramifié, alcényle en C₃-C₆, ramifié ou non ramifié, de préférence allyle, alcoxy en C₁-C₆, (alkyle en C₁-C₄)OC(O)O-, OH, CF₃, ou ensemble un système cyclique à un ou deux noyaux condensé, qui peut être totalement ou partiellement conjugué et qui peut éventuellement comporter un ou deux hétéroatomes du groupe de l'oxygène, du soufre et de l'azote, qui peuvent être identiques ou différents, qui peut éventuellement être substitué par OH, alcoxy en C₁-C₄, alkyle en C₁-C₄ ;
R₅ peut représenter l'hydrogène, aryle, de préférence phényle, -O-phényle, -CR₇R₈-phényle, -CO-phényle, -SO₂-phényle, -CH(OH)-phényle, où le cycle phényle peut être substitué par OH, alcoxy en C₁-C₄, ou -C(O)NR₉R₁₀ ;
R₆ peut représenter l'hydrogène, alcoxy en C₁-C₆-carbonyle, (alkyle en C₁-C₅)-carbonyle ou -C(O)-O-(alkylène en C₁-C₆)-NR₁₁R₁₂ ;
R₇ et R₈, qui peuvent être identiques ou différents, peuvent représenter l'hydrogène, alkyle en C₁-C₈, ramifié ou non ramifié ;
R₉ et R₁₀, qui peuvent être identiques ou différents, peuvent représenter indépendamment l'un de l'autre l'hydrogène, alkyle en C₁-C₈, ramifié ou non ramifié ;
R₁₁ et R₁₂, qui peuvent être identiques ou différents, peuvent représenter indépendamment l'un de l'autre l'hydrogène, alkyle en C₁-C₈, ramifié ou non ramifié ;
éventuellement sous forme des différents isomères optiques, de mélanges des différents énantiomères ou de racémates ainsi que sous forme des bases libres ou des sels d'addition d'acide correspondants avec des acides pharmacologiquement acceptables.

2. Composés de formule générale 1 selon la revendication 1 où
A peut représenter -OCH₂CH₂O-, -CH₂O-, -CH₂CH₂CH₂O-;
R₁ peut représenter alkyle en C₁-C₅, ramifié ou non ramifié, allyle ;
R₂ peut représenter l'hydrogène ;
R₃ peut représenter l'hydrogène, alkyle en C₁-C₆, OCH₃, C₂H₅OC(O)O-, OH, Cl, F, CF₃ ;
R₄ peut représenter l'hydrogène, -OCH₃, OH ;
R₃ et R₄ peuvent représenter ensemble un cycle benzénique condensé, un système 3,4-dihydrocoumarine ou un système 1,3-dioxolane ; qui peut être substitué par OH, alkyle en C₁-C₃, OCH₃ ;
R₅ peut représenter l'hydrogène, phényle, O-phényle, -CR₇R₈-phényle, où le cycle phényle peut être substitué par OH, OCH₃, ou -C(O)NR₉R₁₀ ;
R₆ peut représenter l'hydrogène, alcoxy en C₁-C₄-carbonyle ou -C(O)-O-(CH₂)₂-N(C₂H₅)₂ ;
R₇ et R₈, qui peuvent être identiques ou différents, peuvent représenter indépendamment l'un de l'autre l'hydrogène, CH₃ ou CF₃ ;
R₉ et R₁₀, qui peuvent être identiques ou différents, peuvent représenter l'hydrogène, alkyle en C₁-C₈, ramifié ou non ramifié ;
éventuellement sous forme des différents isomères optiques, de mélanges des différents énantiomères ou de racémates ainsi que sous forme des bases libres ou des sels d'addition d'acide correspondants avec des acides pharmacologiquement acceptables.

3. Procédé de préparation de composés de formule générale 1 **caractérisé en ce que** l'on réduit de manière connue en soi une amidoxime de formule générale 2 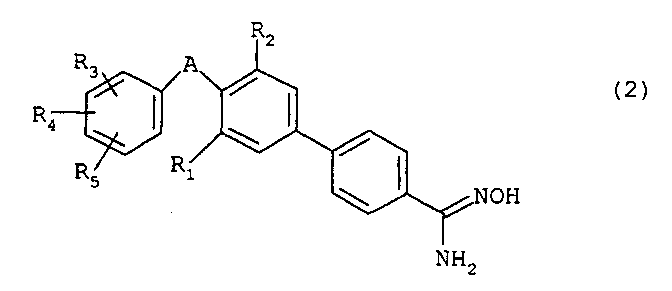 où R₁ à R₅ et A ont la signification indiquée dans la revendication 1.

4. Procédé selon la revendication 3 **caractérisé en ce que** l'on conduit la réduction par voie catalytique, de préférence en présence de nickel de Raney.

5. Procédé selon la revendication 3 ou 4 **caractérisé en ce que** la réduction catalytique de l'amidoxime est conduite dans un alcool inférieur, de préférence dans le méthanol.

6. Procédé de préparation de composés de formule générale 1 **caractérisé en ce que** l'on fait réagir avec l'ammoniac 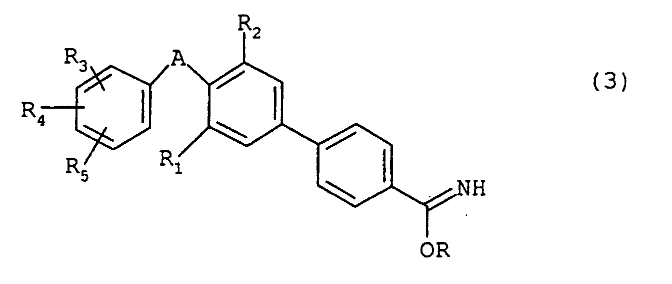 où R₁ à R₅ et A ont la signification indiquée dans la revendication 1.

7. Procédé selon la revendication 6 **caractérisé en ce que** la réaction a lieu dans un solvant organique à des températures entre environ 0°C et la température d'ébullition du mélange réactionnel, de préférence dans un intervalle de température entre la température ambiante et environ 100°C ou la température d'ébullition, dans la mesure où celle-ci est plus basse.

8. Procédé selon la revendication 6 ou 7 **caractérisé en ce que** la réaction a lieu dans un solvant polaire, de préférence dans le méthanol, l'éthanol ou dans un propanol.

9. Procédé de préparation de composés de formule générale 1 **caractérisé en ce que** l'on fait réagir un phénol de formule générale 4 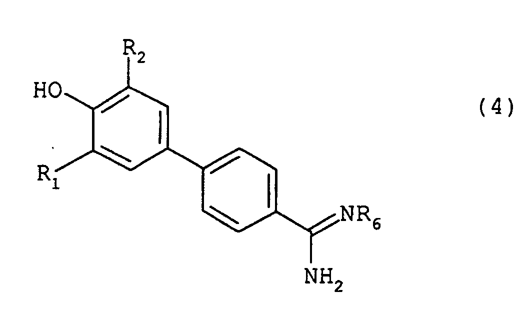 où R₁, R₂, R₆ ont la signification indiquée dans la revendication 1, avec un composé de formule 5 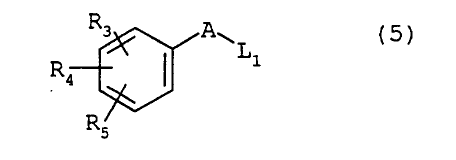 où A, R₃, R₄, R₅ ont la signification indiquée dans la revendication 1 et L₁ représente un groupe partant nucléofuge, de préférence un atome d'halogène ou un reste d'acide sulfonique, dans un solvant aprotique.

10. Procédé selon la revendication 9 **caractérisé en ce que** le solvant est le diméthylsulfoxyde, le diméthylformamide, l'acétonitrile ou un alcool, de préférence le méthanol, l'éthanol ou un propanol.

11. Procédé selon la revendication 9 ou 10 **caractérisé en ce que** la réaction est conduite en présence d'une base, de préférence en présence d'un carbonate de métal alcalin ou alcalino-terreux, d'un hydroxyde métallique ou d'un hydrure métallique.

12. Procédé selon l'une des revendications 9 à 11 **caractérisé en ce que** la réaction est conduite à une température dans un intervalle entre 0°C et le point d'ébullition du mélange réactionnel et de préférence dans un intervalle de température de 0 à 140°C.

13. Procédé de préparation de composés de formule générale 1 **caractérisé en ce que** l'on fait réagir une amidine de formule générale 6 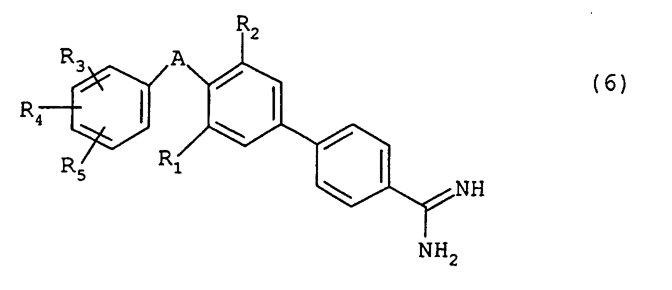 où R₁ à R₅ et A ont la signification indiquée dans la revendication 1, avec un composé de formule générale 7
L₂-R₆' (7)
où
R₆' représente alcoxy en C₁-C₆-carbonyle ou acyle en C₁-C₆ et
L₂ représente un groupe partant nucléofuge, de préférence le chlore, le brome ou acyloxy.

14. Procédé selon la revendication 13 **caractérisé en ce que** la réaction a lieu dans un solvant polaire.

15. Procédé selon la revendication 14 **caractérisé en ce que** le solvant est le tétrahydrofurane, le chlorure de méthylène, le chloroforme ou le diméthylformamide.

16. Procédé selon la revendication 14 ou 15 **caractérisé en ce que** la réaction a lieu en présence d'une base inorganique, de préférence le carbonate de sodium, le carbonate de potassium ou la lessive de soude, ou en présence d'une base organique tertiaire, de préférence la triéthylamine, la N-éthyldiisopropylamine, la N-méthylmorpholine ou la pyridine.

17. Procédé selon l'une des revendications 14 à 16 **caractérisé en ce que** la réaction est conduite dans un intervalle de température entre -30 et 100°C, de préférence dans un intervalle de température entre -10 et 80°C.

18. Préparation pharmaceutique **caractérisée par** une teneur en un composé selon l'une des revendications 1 à 2 et en ses sels d'addition d'acide, outre des adjuvants et supports courants.

19. Composé de formule (1) selon l'une des revendications 1 à 2 destiné à être utilisé comme médicament.

20. Utilisation de composés de formule (1) selon l'une des revendications 1 à 2 pour la production d'un médicament à effet antagoniste de LTB₄.

21. Utilisation de composés de formule générale 1, de leurs stéréoisomères ainsi que de leurs sels d'addition d'acide pour la production d'un médicament pour le traitement thérapeutique de l'arthrite, de l'asthme, d'une bronchopneumopathie chronique obstructive comme la bronchite chronique, du psoriasis, de la colite ulcéreuse, de la gastro- ou entéropathie induite par des antiphlogistiques non stéroïdiens, de la fibrose kystique, de la maladie d'Alzheimer, du choc, des lésions de reperfusion/ischémies, de l'athérosclérose, de la sclérose en plaques.
